# EUROPEAN PATENT APPLICATION

(11) **EP 2 186 821 A1**
(43) Date of publication of application: **19.05.2010**
(21) Application number: 08019676.9
(22) Date of filing: 11.11.2008
(51) Int. Cl.: C07K 14/47, C12N 15/12, A61K 48/00, A61K 38/17

(54) **Protein with promoting effects for axonal growth of neurons of central nervous system**

(71) Applicant: Universität Ulm, 89081 Ulm (DE)
(72) Inventor: Fischer, Dietmar, 89160 Ulm (DE)
(74) Representative: Pfenning, Meinig & Partner GbR

(57) **Abstract**

This invention comprises a polypeptide, a recombinant vector, a recombinant organism as well as RNA- and DNA-sequences. Furthermore, the use of polypeptides and recombinant vectors is described. Additionally the invention comprises methods for medical treatment.

## Description

This invention comprises a polypeptide, a recombinant vector, a recombinant organism as well as RNA- and DNA-sequences. Furthermore, the use of polypeptides and recombinant vectors is described. Additionally the invention comprises methods for medical treatment.

Reciting the state of the art it is known yet, that neurons of the central nervous system (CNS) are normally unable to regenerate injured axons, which severely limits functional recovery after traumatic injury, stroke, or certain neurodegenerative diseases. Regenerative failure has been attributed in part to inhibitory factors associated with CNS myelin, the scar that forms at the lesion site and to an insufficient intrinsic capability of mature neurons to regrow axons (McKerracher L, David S, Jackson DL, Kottis V, Dunn RJ, Braun PE (1994) Identification of myelin-associated glycoprotein as a major myelin-derived inhibitor of neurite growth. Neuron 13:805-811; Chen MS, Huber AB, van der Haar ME, Frank M, Schnell L, Spillmann AA, Christ F, Schwab ME (2000) Nogo-A is a myelin-associated neurite outgrowth inhibitor and an antigen for monoclonal antibody IN-1. Nature 403:434-439; GrandPre T, Nakamura F, Vartanian T, Strittmatter SM (2000) Identification of the Nogo inhibitor of axon regeneration as a Reticulon protein. Nature 403:439-444; Wang KC, Koprivica V, Kim JA, Sivasankaran R, Guo Y, Neve RL, He Z (2002) Oligodendrocyte-myelin glycoprotein is a Nogo receptor ligand that inhibits neurite outgrowth. Nature 417:941-944; Fischer D, He Z, Benowitz LI (2004a) Counteracting the Nogo receptor enhances optic nerve regeneration if retinal ganglion cells are in an active growth state. J Neurosci 24:1646-1651; Fischer D, Petkova V, Thanos S, Benowitz LI (2004b) Switching mature retinal ganglion cells to a robust growth state in vivo: gene expression and synergy with RhoA inactivation. J Neurosci 24:8726-8740).

Retinal ganglion cells (RGCs) are typical projection neurons of the CNS and are therefore also insufficient to regenerate axons after optic nerve injury. Instead, more than 90% of RGCs undergo apoptosis a few days after an intraorbital optic nerve crush (Berkelaar M, Clarke DB, Wang YC, Bray GM, Aguayo AJ (1994) Axotomy results in delayed death and apoptosis of retinal ganglion cells in adult rats. J Neurosci 14:4368-4374).

Nevertheless, intravitreal application of lental crystallins, their release from the injured lens or alternatively intravitreal injection of zymosan protect RGCs from axotomy induced apoptosis and switch these neurons into a robust regenerative state (Fischer D, Pavlidis M, Thanos S (2000) Cataractogenic lens injury prevents traumatic ganglion cell death and promotes axonal regeneration both in vivo and in culture. Invest Ophthalmol Vis Sci 41:3943-3954; Leon S, Yin Y, Nguyen J, Irwin N, Benowitz LI (2000) Lens injury stimulates axon regeneration in the mature rat optic nerve. J Neurosci 20:4615-4626; Fischer D, Heiduschka P, Thanos S (2001) Lens-injury-stimulated axonal regeneration throughout the optic pathway of adult rats. Exp Neurol 172:257-272; Fischer D, Hauk T, Müller A, Thanos S (2008) Crystallins of the beta/gamma-superfamily mimic the effects of lens injury and promote axon regeneration. Molecular Cellular Neuroscience doi:10.1016/j.mcn.2007.11.-002).

In this state RGCs can regrow injured axons at higher growth rates from retinal explants, regenerate up to 40% of axons into an peripheral nerve graft and even distal to the lesion site in the inhibitory milieu of the optic nerve (Fischer D, Pavlidis M, Thanos S (2000) Cataractogenic lens injury prevents traumatic ganglion cell death and promotes axonal regeneration both in vivo and in culture. Invest Ophthalmol Vis Sci 41:3943-3954; Fischer D, Heiduschka P, Thanos S (2001) Lens-injury-stimulated axonal regeneration throughout the optic pathway of adult rats. Exp Neurol 172:257-272).

Astrocyte-derived ciliary neurotrophic factors (CNTF) whose expression is upregulated and continuously released after the before mentioned treatments is one of the major contributing factors mediating this phenomenon (Muller A, Hauk TG, Fischer D (2007) Astrocyte-derived CNTF switches mature RGCs to a regenerative state following inflammatory stimulation. Brain 130:3308-3320; Hauk TG, Müller A, Lee J, Schwendener R, Fischer D. Neuroprotective and axon growth promoting effects of intraocular inflammation do not depend on oncomodulin or the presence of large numbers of activated macrophages. Exp Neurol. 2008 Feb; 209(2):469-82. Epub 2007 Sep 29; Fischer D. CNTF, a key factor mediating the benefical effects of inflammatory reactions in the eye. Brain. 2008 Jun; 131 (Pt 6):e97. Epub 2008 Feb 20.).

The switch of RGCs into the active regenerative state is associated with a dramatic change in gene expression (Fischer D, Petkova V, Thanos S, Benowitz LI (2004b) Switching mature retinal ganglion cells to a robust growth state in vivo: gene expression and synergy with RhoA inactivation. J Neurosci 24:8726-8740) .

Next to several known genes that had previously been associated with axon regeneration several expressed sequence tags (ESTs), representing genes whose function has not yet been explored, were differentially expressed in regenerating RGCs.

The problem for the present invention is that until now no method as well as medical treatment or medicine is available to treat diseases regarding the nervous system which sufficiently stimulate axon growth of injured neurons and delay apoptosis. Thus, a treatment is needed which can induce regeneration or growth of e.g. neurons and protect these neurons from cell death.

This problem is partially solved by the use of a polypeptide according to claim 1. Claims 3 and 4 describe further use of a polypeptide according to the present invention and claim 5 the use of a recombinant vector. For solving the problem claim 14 offers a inventive RNA- and claim 15 a inventive DNA-nucleotide sequence, furthermore, claim 16 specifies a polypeptide which is produced by using the previous described RNA or DNA and claim 17 is regarding a rat polypeptide with a specified amino acid sequence. For solving the problem claim 18 of the invention refers to a recombinant vector, claim 20 to a recombinant organism and claim 21 refers to a method of a medical treatment. Advantageous developments of the invention are described respectively in the dependent claims.

In the invention the identification of a so far uncharacterized ring finger protein (inventive protein) is reported. Overexpression of the inventive protein promotes neurite outgrowth in PC12 cells (pheochromocytoma of a rat adrenal medulla) and gene therapeutic overexpression spurs axon regeneration of RGCs *in vitro* and *in vivo.*

Upregulation of the inventive protein in RGCs was confirmed on RNA and protein level and also found in differentiating PC12 cells. Overexpression of the inventive protein enhanced NGF-induced neurite outgrowth of PC12 cells. Adeno-associated virus mediated overexpression of the inventive protein in RGCs improved survival of RGCs and enhanced axonal regeneration from retinal explants.

In vivo overexpression of the inventive protein allowed transfected RGCs to regenerate axons beyond the lesion site of the crushed optic nerve. These data strongly suggest that the inventive protein is playing an important role in axon regeneration in RGCs.

Overexpression of the inventive protein promotes neurite outgrowth in PC12 cells and gene therapeutic overexpression spurs axon regeneration of RGCs *in vitro* and *in vivo.*

The invention comprises the use of a polypeptide comprising at least the ring finger domain of RING finger protein 122, of a recombinant vector comprising DNA or RNA coding at least for the ring finger domain of RNF 122 and/or of a recombinant organism comprising DNA or RNA coding at least for the ring finger domain of RNF 122 in medicine.

Preferred is the use of a polypeptide comprising at least the ring finger domain of RING finger protein 122, of a recombinant vector comprising DNA or RNA coding at least for the ring finger domain of RNF 122 and/or of a recombinant organism comprising DNA or RNA coding at least for the ring finger domain of RNF 122 as a medicinal drug.

A further preferred embodiment is the use of a polypeptide comprising at least the ring finger domain of RING finger protein 122, of a recombinant vector comprising DNA or RNA coding at least for the ring finger domain of RNF 122 and/or of a recombinant organism comprising DNA or RNA coding at least for the ring finger domain of RNF 122 for manufacturing of a medicinal drug for treatment of at least one of the diseases of the group comprising diseases of the nervous system, in particular of the central nervous system; ocular diseases, in particular retinal diseases, glaucoma, intraocular inflammation, optic nerve injuries, in particular crushed optic nerves; neuronal diseases, traumatic injuries of the nervous system; stroke; diseases of spinal cord and diseases after an apoplexy.

Additionally, use of a polypeptide comprising at least the ring finger domain of RING finger protein 122, of a recombinant vector comprising DNA or RNA coding at least for the ring finger domain of RNF 122 and/or of a recombinant organism comprising DNA or RNA coding at least for the ring finger domain of RNF 122 for manufacturing of a medicinal drug for treatment of ocular diseases by intravitreal injection of the medicinal drug is described. This method allows a direct application to the organism, where the medicinal drug is needed. So it is possible to use low doses and herewith the probability for appearing of side effects is reduced.

Preferred is the use of a recombinant vector comprising DNA or RNA coding at least for the ring finger domain of RNF 122 and/or of a recombinant organism comprising DNA or RNA coding at least for the ring finger domain of RNF 122 according to one of the previous claims, **characterized in that** the recombinant vector and/or the recombinant organism comprises DNA or RNA of mouse origin or DNA or RNA of rat origin or DNA or RNA of human origin. Depending on further application the most suitable possibility can be used.

An other preferred embodiment comprises the use of a recombinant vector comprising DNA or RNA coding at least for the ring finger domain of RNF 122 and/or of a recombinant organism comprising DNA or RNA coding at least for the ring finger domain of RNF 122 as described previously, **characterized in that** the recombinant vector and/or the recombinant organism comprises at least the following RNA nucleotide sequence coding for at least the ring finger domain of RNF 122:

acacgucaaacagaccuucugaag
uuccccuuccuacuugauccacacgaaggc
acggucguacggaaaguggcguucacagac
cacuucaccgaucuucacgcaacgcagacg
ggguacacg.

Furthermore, the use of a recombinant vector comprising DNA or RNA coding at least for the ring finger domain of RNF 122 and/or of a recombinant organism comprising DNA or RNA coding at least for the ring finger domain of RNF 122 according to claim 6, **characterized in that** the recombinant vector and/or the recombinant organism comprises at least the following RNA nucleotide sequence: 
uacgugguaaggucaccacauugcccaca
aagacaccggaccccgaccacucaugguug
uucaggacgaguuacgguguuagucaaag
guccuggaaggggaguuguagauguaccag
uagaagccgugaccguagaaacagaaauac
gagucagaguagaagacgacgaugaaguag
ucguuugaggccuugguccgugucucgcuc
gcuaugccgauguuccuccaccacgaauuu
ccacuacgauucuucaaugucgagauaccc
gucugg acacgucaaacagaccuucugaag
uuccccuuccuacuugauccacacgaaggc
acggucguacggaaaguggcguucacagac
cacuucaccgaucuucacgcaacgcagacg
ggguacacg uuguucggguaacgaccgggg
ugccucuggagcgucucguaacccuaggac
gaccuacuuaaccacauu
 is preferred.

An other embodiment describes the use of a recombinant vector comprising DNA or RNA coding at least for the ring finger domain of RNF 122 and/or of a recombinant organism comprising DNA or RNA coding at least for the ring finger domain of RNF 122 as described previously, wherein the recombinant vector and/or the recombinant organism comprises at least the following DNA nucleotide sequence:

tgtgcagtttgtctggaagacttc
aaggggaaggatgaactaggtgtgcttccg
tgccagcatgcctttcaccgcaagtgtctg
gtgaagtggctagaagtgcgttgcgtctgc
cccatgtgc.

The use of a recombinant vector comprising DNA or RNA coding at least for the ring finger domain of RNF 122 and/or of a recombinant organism comprising DNA or RNA coding at least for the ring finger domain of RNF 122 according to claim 8, **characterized in that** the recombinant vector and/or the recombinant organism comprises at least the following DNA nucleotide sequence: 
atgcacccattccagtggtgtaacgggtgt
ttctgtggcctggggctggtgagtaccaac
aagtcctgctcaatgccaccaatcagtttc
caggaccttcccctcaacatctacatggtc
atcttcggcactggcatctttgtctttatg
ctcagtctcatcttctgctgctacttcatc
agcaaactccggaaccaggcacagagcgag
cgatacggctacaaggaggtggtgcttaaa
ggtgatgctaagaagttacagctctatggg
cagacc tgtgcagtttgtctggaagacttc
aaggggaaggatgaactaggtgtgcttccg
tgccagcatgcctttcaccgcaagtgtctg
gtgaagtggctagaagtgcgttgcgtctgc
cccatgtgc aacaagcccattgctggcccc
 
acggagacctcgcagagcattgggatcctg
ctggatgaattggtgtaa
 is comprised by an other preferred embodiment of the invention.

It is possible to use a polypeptide as described above, wherein the polypeptide comprises at least or consists of the following amino acid sequence:
CAVCLEDF KGKDELGVLP CQHAFHRKCL VKWLEVRCVR PMC.

Further preferred is the use of a polypeptide as described before, which comprises or consists of the following amino acid sequence: 
MHPFQWCNGC FCGLGLVSTN KSCSMPPISF QDLPLNIYMV
IFGTGIFVFM LSLIFCCYFI SKLRNQAQSE RYGYKEVVLK
GDAKKLQLYG QTCAVCLEDF KGKDELGVLP CQHAFHRKCL
VKWLEVRCVR PMCNKPIA*P *E**Q*IGIL LDELV,
 wherein * indicates a not specified amino acid.

These amino acids are preferably chosen as follows: as amino acid 139 G or S *and*/*or* as amino acid 141 T or S *and*/*or* as amino acid 143 T or A *and*/*or* as amino acid 144 S or T *and*/*or* as amino acid 146 S or N. The mentioned combinations show excellent properties for application and in effectiveness.

Further the use of a polypeptide comprising at least the ring finger domain of RING finger protein 122, of a recombinant vector comprising DNA or RNA coding at least for the ring finger domain of RNF 122 and/or of a recombinant organism comprising DNA or RNA coding at least for the ring finger domain of RNF 122 as described previously, **characterized in that** the polypeptide, the recombinant vector and/or the recombinant organism is used in combination with a nerve growth factor (NGF) and/or axon growth promoting factors is a preferred modification of the invention. The combination of the before mentioned substances allow a higher variety in treatment of diseases, preferably of the nervous system, and therefore allow an individual adaptation to given factors.

The invention comprises RNA with the following nucleotide sequence:

uacgugguaaggucaccacauugcccaca
aagacaccggaccccgaccacucaugguug
uucaggacgaguuacgugguuagucaaag
guccuggaaggggaguuguagauguaccag
uagaagccgugaccguagaaacagaaauac
gagucagaguagaagacgacgaugaaguag
ucguuugaggccuugguccgugucucgcuc
gcuaugccgauguuccuccaccacgaauuu
ccacuacgauucuucaaugucgagauaccc
gucugg acacgucaaacagaccuucugaag
uuccccuuccuacuugauccacacgaaggc
acggucguacggaaaguggcguucacagac
cacuucaccgaucuucacgcaacgcagacg
ggguacacg uuguucggguaacgaccggg
ugccucuggagcgucucguaacccuaggac
gaccuacuuaaccacauu and DNA with the following nucleo-
tide sequence:
atgcacccattccagtggtgtaacgggtgt
ttctgtggcctggggctggtgagtaccaac
aagtcctgctcaatgccaccaatcagtttc
caggaccttcccctcaacatctacatggtc
atcttcggcactggcatctttgtctttatg
ctcagtctcatcttctgctgctacttcatc
agcaaactccggaaccaggcacagagcgag
cgatacggctacaaggaggtggtgcttaaa
ggtgatgctaagaagttacagctctatggg
cagacc tgtgcagtttgtctggaagacttc
aaggggaaggatgaactaggtgtgcttccg

tgccagcatgcctttcaccgcaagtgtctg
gtgaagtggctagaagtgcgttgcgtctgc
cccatgtgc aacaagcccattgctggcccc
acggagacctcgcagagcattgggatcctg
ctggatgaattggtgtaa.

In a preferred embodiment the inventive polypeptide is produced by using the previous described RNA or DNA.

Furthermore, a rat polypeptide comprising or consisting of the following amino acid sequence:

MHPFQWCNGC FCGLGLVSTN KSCSMPPISF QDLPLNIYMV
IFGTGIFVFM LSLIFCCYFI SKLRNQAQSE RYGYKEVVLK
GDAKKLQLYG QTCAVCLEDF KGKDELGVLP CQHAFHRKCL
VKWLEVRCVR PMCNKPIAGP TETSQSIGIL LDELV is preferen-
tial.

In another aspect of the invention the recombinant vector contains RNA or DNA or DNA or RNA which codes for polypeptides described previously.

The recombinant vector can be a plasmid, whereas an AAV-expression vector is preferred.

The Recombinant organism according to an embodiment of the invention is **characterized in that** it has at least one of the following characteristics a) to d)
a) it has been transformed with a vector as described previously;
b) it comprises at least a sequence of DNA or RNA which codes for a polypeptide as described previously;
c) it is a virus, preferably an adeno associated virus (AAV); and
d) it consists of cells, preferably of human embryonic cells (HEC), pheochromocytoma cells (PC12) or retinal ganglion cells (RGC).

Organisms as described allow a vast variety of application, which can be optimized according to parameters given by the surrounding.

The invention comprises additionally a method of a medical treatment, **characterized in that** a polypeptide comprising at least the ring finger domain of RING finger protein 122, a recombinant vector comprising DNA or RNA coding at least for the ring finger domain of RNF 122 and/or a recombinant organism comprising DNA or RNA coding at least for the ring finger domain of RNF 122 is applied to an animal, in particular a mammal, in particular a human being.

These methods can be used for the treatment of at least one of the diseases of the group comprising diseases of the nervous system, in particular of the central nervous system; ocular diseases, in particular retinal diseases, intraocular inflammation, optic nerve injuries, in particular glaucoma; neuronal diseases, in particular injured axons; traumatic injuries of the nervous system; stroke; diseases of spinal cord and diseases after an apoplexy.

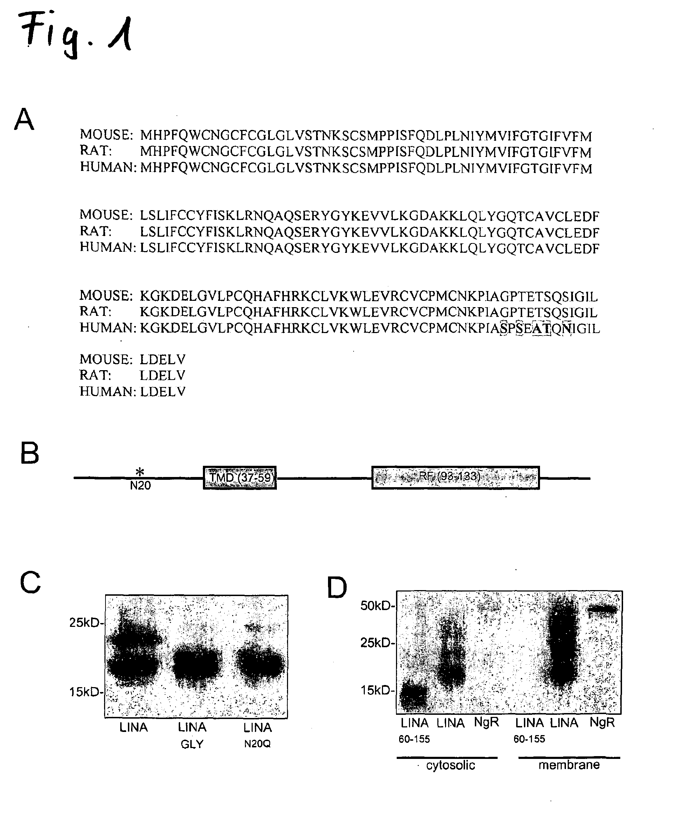

The following example 1 and figures 1 to 7 are intended to describe an example of the invention in more detail without reducing it thereto.

### Example 1:

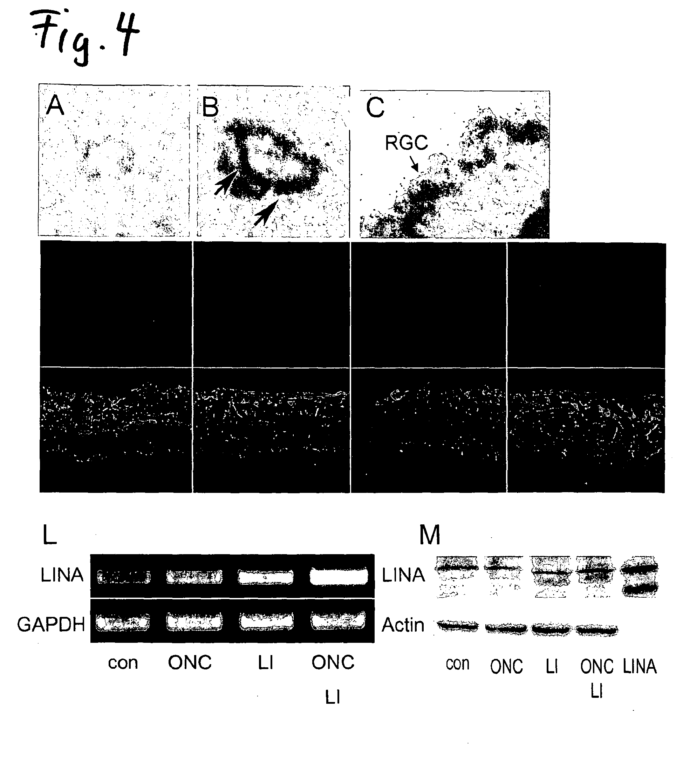

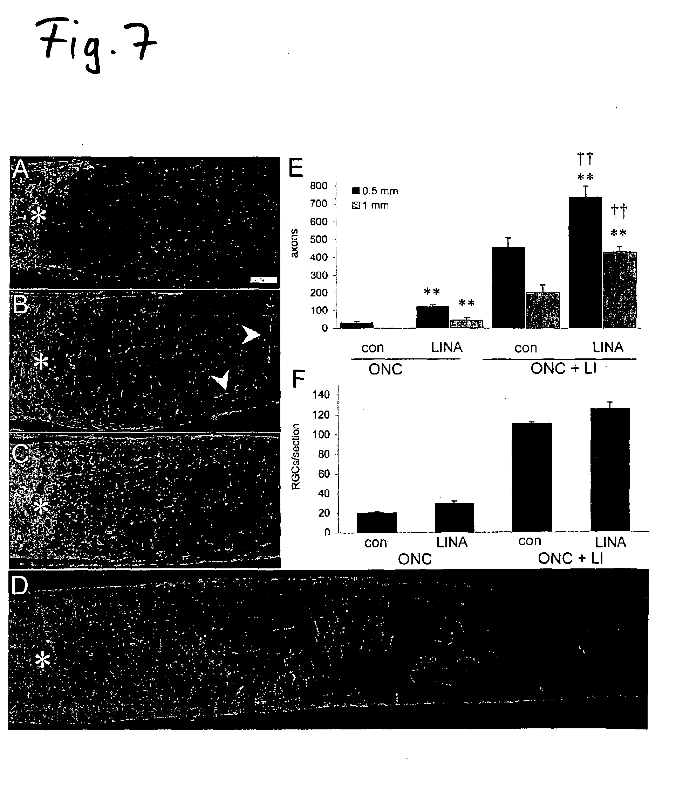

In the following one example of the inventive protein, which in the following is called "LINA" (lens injury induced factor with axon growth promoting activity), is described with respect to the following figures:
Figure 1 shows LINA, a ring finger protein according to the invention and its predicted domains.
   A) Amino acid sequence of mouse/rat and human LINA (HS; homo sapience). The sequence of LINA is highly conserved mainly from transmembrane domain to ring finger domain. The sequence of mouse and rat is identical and there is five amino acids difference with the sequence of human LINA at C-terminus. The sequence of LINA is highly conserved and differences to the human amino acid sequence are restricted to the c-terminus (bold letters) of the protein. B) Schematic drawing of LINA with its functional and structural domains. C) Western blot: Exogenous expressed HA-tagged LINA runs as two bands at 19 and 23 kD in gels. Treatment with glycosidase (PNGaseF) or a mutation of asparagine to glutamine at position 20 eliminated the upper band, demonstrating that LINA is glycosylated at N20. D) HA-tagged LINA is strongly accumulated in the membranous fraction, whereas a mutant form lacking the putative transmembrane domain is found in the cytosolic fraction. As a control the membrane bound HA-tagged Nogo-receptor, which was mainly found in the membrane fraction, was used.

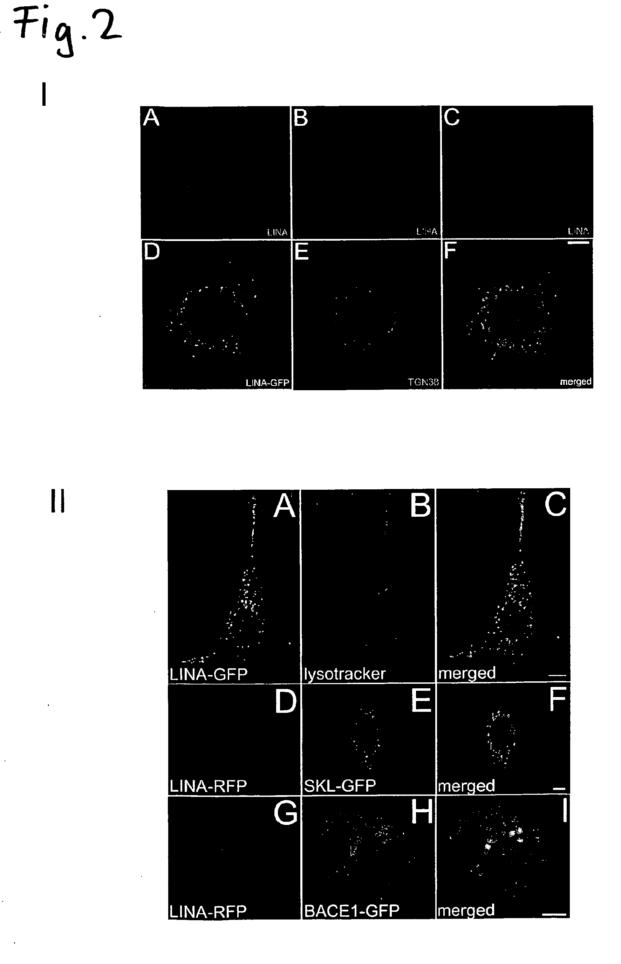

Figure 2 shows intracellular localisation of LINA.
   I: A) Endogenous LINA detected by an affinity purified anti-LINA antibody in a hippocampal neuron derived from an embryonic rat (E20) and cultured for 2 weeks. LINA shows a vesicular staining pattern of the cell body and processes and accumulates in the Trans-Golgi Network (TGN). B) Endogenous expressed LINA in cultured RGCs. LINA staining is found in the cell body and to a lower extent in the outgrowing axon. C) Exogenous expressed HA-tagged LINA in a PC12 cell. D-F) PC12 cell expressing a LINA-GFP-fusion protein. E) Same cell in D but stained with an antibody against TGN38 a marker for the TGN. F) Merged picture of D and E.
   II: A-C) PC12 cell expressing a LINA-GFP-fusion protein. B) Same cell in A stained with lysotracker, a marker for lysosomes. C) Merged picture of A and B. D-F) PC12 cell expressing a LINA-RFP-fusion protein. E) Same cell expressing a SKL-GFP-fusion protein, with a signal sequence for peroxisome. F) Merged picture of D and E. G-I) PC12 cell expressing a LINA-RFP-fusion protein. H) Same cell expressing a BACE1-GFP-fusion protein, a typical secretoric vesicle protein. I) Merged picture of G and H.

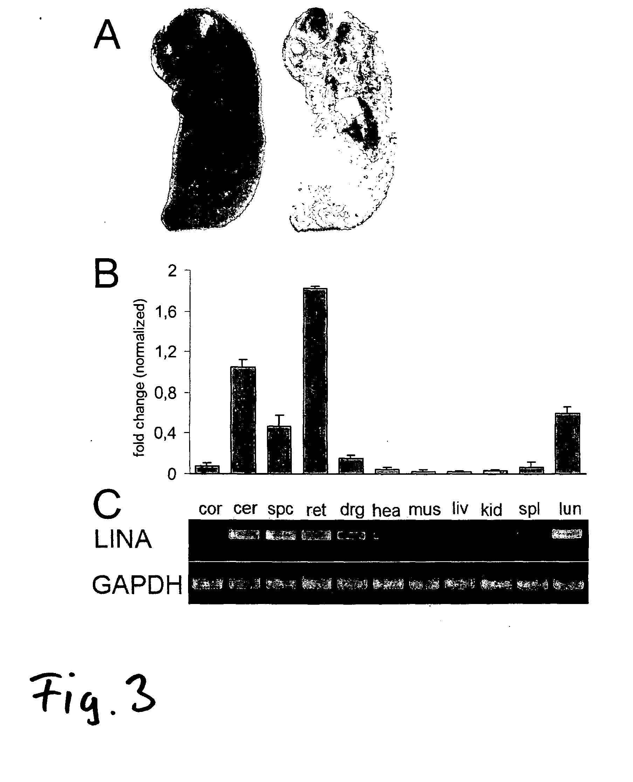

Figure 3 shows LINA expression in embryonic and adult tissue.
   I: A) In-situ-hybridisation of rat embryo (E18). LINA was detected in retina (ret), cerebellum (cer), the subventricular zone (svz) in the brain and lung (lun) .
   B) Quantitative real-time-PCR was used to measure relative LINA expression in various tissues of the adult rat. LINA expression was high in retina, cerebellum and lung; moderate levels were detected in spinal cord (spc), brain cortex (cor) and dorsal root ganglia (DRG); very low levels were found in heart (hea), muscle (mus), liver (liv), kidney (kid) and spleen (spl).
   C) RT-PCR confirmed results of quantitative real-time-PCR
Figure 4 shows differentially expressed LINA in regenerating RGCs.
   A-C) *In situ*-hybridisation of naive and LI treated retina. LINA is detected in retinas 5 days after ONC and LI (A), whereas naive retina shows a weak signal (B). C) As demonstrated by dipped sections most of the signal of a LI treated retina is derived from cells in the ganglion cell layer.
   D-K) Immunohistochemical staining of retinas after various treatments.
   D-G) Staining with an affinity purified anti-LINA antibody, H-K) staining with a βIII-tubulin antibody to detect RGCs and merged pictures. D, H) naive retia; E, I) retina subjected to ONC; F, J) retina exposed to LI and G, K) retina subjected to ONC + LI.
   L) RT-PCR: LINA is expressed in retina. Expression levels are slightly upregulated after ONC and further upregulated after additional LI or LI alone.
   M) Western-blot of retinas treated as mentioned before but sampled after 5 days. The LINA antibody detected endogenous LINA at 19 kD at the same level as the upper band of exogenously expressed LINA in HEK293 cells, suggesting that endogenous LINA exists in the glycosylated form only. Levels of GAPDH verified that similar amounts of LINA were loaded on the gel.

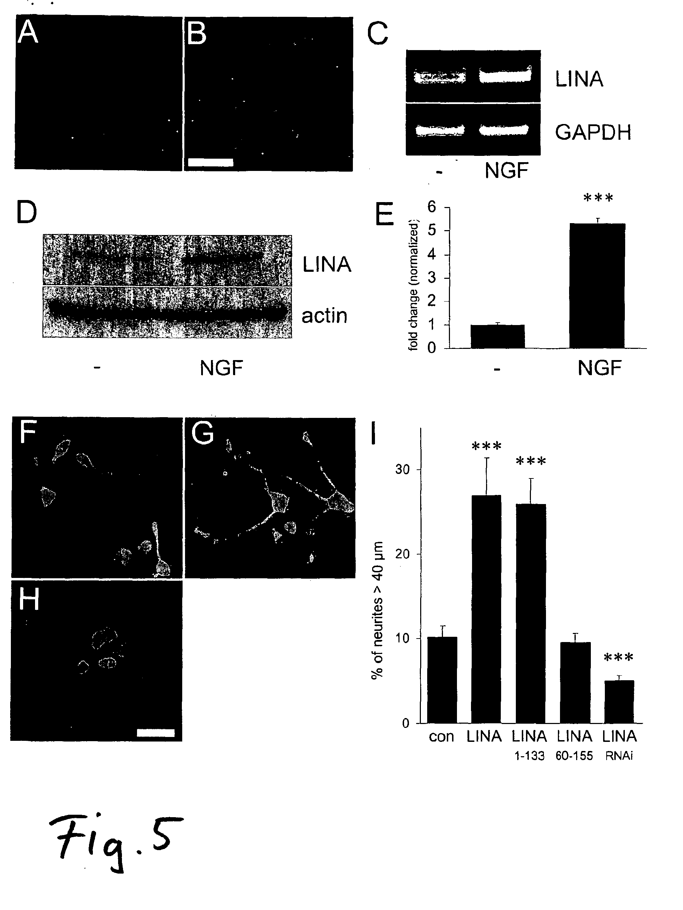

Figure 5 shows LINA essentiality for neurite outgrowth and that its overexpression enhances neurite outgrowth in PC12 cells.
   A-E) Endogenous LINA expression in PC12 cells. NGF differentiated PC12 cells show increased LINA expression. A) PC12 cells without being exposed to NGF stained by antibody LINA. B) PC12 cells treated with NGF stained by antibody LINA. C) Expression level of LINA in PC12 cells assessed by RT-PCR. D) Expression level of LINA in PC12 cells assessed by western blotting. E) Expression level of LINA in PC12 cells assessed by real-time PCR. NGF treatment increases LINA expression in PC12 cells.
   F-I) Differentiated PC 12 cells were transfected with (F) control plasmids (GFP), (G) plasmids for LINA overexpression, (H) plasmids to express RNAi hairspin constructs (I) Quantitation of neurite outgrowth: LINA and a c-terminus depleted form of LINA overexpression significantly increases neurite outgrowth compared to controls. Decreased LINA expression by RNAi results in significantly less outgrowth compared to controls, suggesting an essential role for endogenous LINA in neurite outgrowth. A depletion of the N-terminus of LINA results in a loss of function (effects of transgene expression outgrowth relative to control *** p < 0.001).

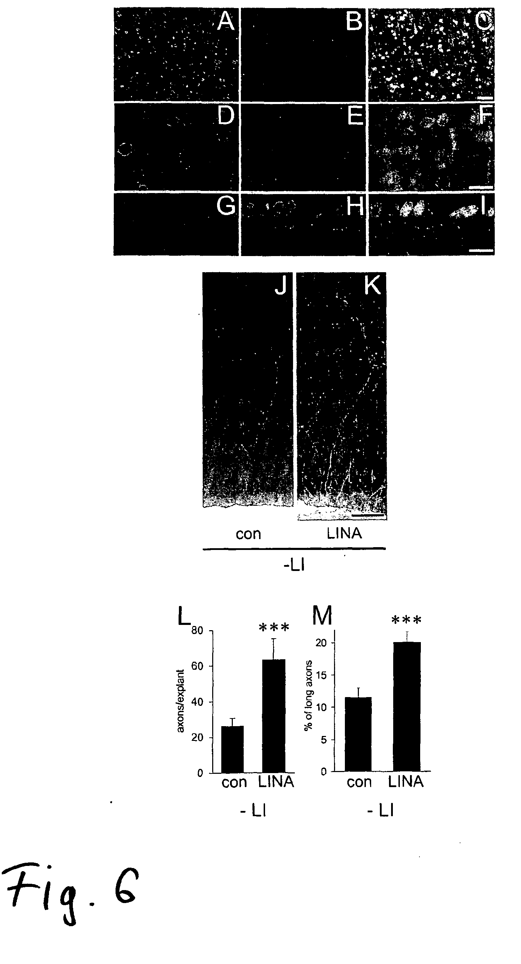

Figure 6 shows the AAV-mediated transfection of RGCs.
   A-C) Flat mounted rat retina three weeks after intravitreal injection with AAV-LINA-HA-IGFP, double stained for GFP to detect transfected cells (A) and βIII-tubulin, a selective marker for RGCs in the retina (B). C) Merged image. D, E, F) Higher magnification of areas from A-C. G) Exogenous expressed LINA-HA is detected in RGCs using an anti-hemagglutinin antibody. H) RGCs stained with an anti-βIII tubulin antibody. I) Merged image. J-M) Retinal explants grown on a permissive PDL/laminin substrate. Control retina (J) and retina transfected with AAV-LINA (K) and not exposed to LI *in vivo.* LINA overexpression enhances axon outgrowth. L,M) Quantitation of axon growth by number and percentage of axons extending 0.5 mm as indicated. ***p < 0.01
   Scale bars: A-C = 50 µm
   D-F = 20 µm
   G-I = 20 µm
Figure 7 shows axon regenerating into the optic nerve.
   A-D) Longitudinal sections through the optic nerve showing GAP43-positive axons distal to the injury site (asterisks) two weeks after surgery with or without LI and transgene expression. A-B) Axon regeneration after transfection with AAV carrying an empty vector (A) or LINA^{WT} without additional LI (B). LINA overexpression results in some axon regrowth distally from the lesion site. C-D) Axon regeneration after transfection with AAV carrying an empty vector (C) or LINA^{WT} and additional LI. The dominant negative form of LINA abrogates LI effects, whereas LINA overexpression combined with LI results in significant stronger regeneration. E) Quantitation of axons regenerating into the optic nerve after various treatments. Total number of axons at 0.5 (light bars) and 1 mm (dark bars) distal to the injury site. F) Cell survival (βIII tubulin-positive RGCs per section). **p < 0.01 increase relative to GFP-transfected controls; ^{††}p < 0.01 decrease relative to GFP transfected controls.
   Scale bar: 100 µm

### METHODS

### Cloning of LINA

The sequences provided on the microarray (probe set ID: 1373485_at, Chip RAE230A) revealed a significant homology to the 3' end of an uncharacterized mouse protein (ring finger protein 122; RNF 122). The sequence of the mouse gene to rat genome databases was blasted. Based on the sequences which were found, primers flanking the putative coding sequence of the corresponding protein were designed. The following primers were used for RT-PCR:
forward: 5'-CATTGATGCACCCATTCCAGTGGTGTAA-3' and
reverse: 5'-GCAAGGAGCGCTTACACCAATTCAT-3'. The PCR products were cloned into a TOPO-cloning vector (Invitrogen). The cDNAs of several clones were sequenced. For cloning the cDNA into a pAAV-IRES-hrGFP expression vector (Stratagene) restriction sites were introduced by PCR using the following primers:
forward: 5'-GTCAAATTGAATTCCCTTGACTCCACTCCTCC-3' and
reverse: 5'-GTTATATTCTCGAGGCAAGGAGCGCTTACACC-3'.
C-terminal HA-tagged LINA was generated by means of the following reverse primer:
5'-ATATTCTCGAGTTAAGCATAATCTGGAACATCATATGGATACATCACCAA TTCATCCAGCAGGAT-3'.

### In situ hybridization

*In situ* hybridization was performed according to previously described protocols (Boeckers TM, Winter C, Smalla KH, Kreutz MR, Bockmann J, Seidenbecher C, Garner CC, Gundelfinger ED (1999) Proline-rich synapse-associated proteins ProSAP1 and ProSAP2 interact with synaptic proteins of the SAPAP/GKAP family. Biochemical & Biophysical Research Communications 264:247-252.). Transcripts encoding LINA were detected with antisense oligonucleotide purchased from Thermo Electron Corporation (Ulm, Germany) directed against the 5' and 3' end of the mRNA: 5'-GCCACTTCACCAGACACTTGCGGTGAAAGGCATGCTGGC-3' and 5'-GCAATGGGCTTGTTGCACATGGGGCAGACGCAACGCAC-3. After hybridization sections were either exposed to Amersham beta-max Hyperfilm for 1-3 weeks or dipped in NTB3 nuclear track emulsion (Kodak; diluted 1:1 with water), stored for 4-8 weeks in the dark at 4 °C, developed and counterstained with cresyl violet.

### Semi-quantitative reverse-transcription polymerase chain reaction (RT-PCR) and Real-time PCR:

Quantitation of LINA was performed with the Quanti-Tect SYBR^{®} Green RT-PCR Kit (Qiagen, No 204243), using a LightCycler Instrument (LightCycler System, Roche Diagnostics, Germany). The amplification signals were detected in real-time permitting accurate quantification of the amounts of the initial RNA template.

Messenger-RNA (100 ng) was reverse transcribed and the cDNA was amplified with specific primers during 50 cycles according to the manufacturer's protocol. All reactions were performed in duplicates and four independent samples (from different eyes) per group were run. Quantitative analysis was performed using the LightCycler Software. The specificity of the PCR products of each run was determined and verified with the LightCycler Software's melting-curve analysis feature. The following primers were used to detect LINA: forward: 5'-CCATTCCAGTGGTGTAACG -3' and reverse: 5'-AGATGCCAG TGCCGAAGA-3'. For GAPDH detection we used custom made primers: GAPDH_RN_Gapd_1 SG QuantiTect^{®} (Qiagen).

For semi-quantitative RT-PCR the following primer sequences were used for LINA:
5'-CATTGATGCACCCATTCCAGTGGTGTAA-3' and
   reverse: 5'-GCAAGGAGCGCTTACACCAATTCAT-3'.
For GAPDH we used the following primers:
   forward: '5-GGCCAAGGTCATCCATGACAACTT-3',
   reverse: '5-CCTGCTTCACCACCTTCTTGATGT-3'.

### Immunohistochemistry

Animals were anesthetized and perfused through the heart with PBS, followed by 4 % paraformaldehyde. Eyes with optic nerves segments attached were dissected free from connective tissue, postfixed overnight, transferred to 30% sucrose overnight (4 °C), and frozen with tissue-tek. Frozen sections were cut on a cryostat, thaw-mounted onto coated glass slides (Superfrost Plus) and stored at -80 °C until additional use. As primary antibody, we used affinity chromatography- purified rabbit anti-LINA antibody at a dilution of 1:50. To visualize RGCs, the monoclonal mouse TUJ1 antibody at a dilution of 1:1000 was used. Secondary antibodies included an anti-rabbit IgG conjugated to Alexa Fluor 594 (1:1000, molecular probes) and anti-mouse IgG conjugated to Alexa Fluor 488 (1:1000, molecular probes). Fluorescent sections were dyed at last with DAPI and covered using mowiol and analyzed under a fluorescent microscope.

### Western-blotting

Rats were killed with 14 % chloral hydrate (i.p.) and their eyeballs were enucleated and dissected. The vitreous body was removed and the retinas were collected in lysis buffer (1 % Triton-X-100, 0.1% SDS) with 1/100 protease inhibitor (Calbiochem, California, USA). Retinas were homogenized and centrifuged at 14,000 rpm for 20 minutes. The supernatant was used for Western blot analysis. Separation of proteins was performed with 12 % sodium-dodecyl-sulfate-polyacrylamide gel electrophoresis (SDS-PAGE) reducing conditions with β-mercaptoethanol according to standard protocols (Bio-Rad, Hercules, USA). After SDS-PAGE proteins were transferred to nitrocellulose membranes (Amersham). The blots were blocked in 5 % dried milk in Tris-buffered saline-Tween 20 (TBS-T) and processed for immunostaining with monoclonal primary antibody against LINA (dilution 1:1000), a monoclonal antibody against β-actin (1:7500) over night at 4°C. Bound antibodies were visualized with an anti-sheep, anti-rabbit or anti-mouse IgG secondary antibodies diluted 1:80,000 (all Sigma, St. Louis, USA). The antigen-antibody complex was detected by enhanced chemoluminescence (ECL, Amersham).

### Membrane fragmentation

HEK293 cells expressing wild type HA-tagged LINA for 24 h were collected in lysis buffer (20 mM Tris-HCl, pH 7.5, 10 mM KC1, 250 mM sucrose, 10 mM NaF, 1 mM DTT, 0.1 mM Na₃VO₄ and 1 mM PMSF & 1/100 protease inhibitor cocktail). The cells were broken by repeated passage through a 21-G needle and after removal of intact cells and nuclei (500 x g, 10 min) sucrose was added to the postnuclear supernatant to 2 M. This was overlaid with 1.7 M and 0.8 M sucrose in 20 mM Tris-HCl (pH 7.5) and centrifuged for 20 h at 130,000 x g, 10 °C. The 1.7/ 0.8 M interface membrane peak was collected, diluted two-fold with 20 mM Tris-HC1, and membranes were pelleted at 150,000 x g for 30 min. After two-fold dilution with 20 mM Tris-HC1 proteins from the soluble 2 M fraction were precipitated with 10 % trichloroacetic acid on ice for 60 min.

### PC12 cell neurite outgrowth assay

PC12 cells were grown on poly-L-lysine in four-well plates (Nunc/Wiesbaden, Germany) in DMEM plus 5 % fetal calf serum FCS (Invitrogen), 10 % heat-inactivated horse serum (Invitrogen), and 100 U penicillin/ streptomycin (Sigma). Cells were transfected by using Lipofectamine 2000 (Invitrogen). After transfection nerv growth factor NGF (Sigma) was added to the medium to initiate the differentiation of cells. After three days in culture pictures of fluorescent cells were taken by means of a fluorescent microscope equipped with a digital camera (Zeiss, Germany). Neurite outgrowth was assessed in 300 consecutive cells per well by an observer blind to the experimental conditions in each well. The length of neurites was measured by using Axiovison software (Zeiss). Neurite outgrowth was defined as the fraction of cells with neurites of more than 37 µm in length averaged over four replicate wells. All results are presented ±SEM and are representative of at least three independent experiments.

### Viral construction

cDNA encoding wild-type LINA (LINA^{WT}) was inserted into AAV-MCS2-IGFP plasmid, kindly provided by Harvard Gene Therapy Initiative. Gene expression was driven by a cytomegalovirus promotor; constructs also expressed enhanced green fluorescent protein (GFP) from an internal ribosomal entry site (IRES). Controls were transfected with viruses expressing GFP alone.

For virus production HEK293 cells were cotransfected with the AAV-MCS2-IGFP plasmid, pHGTI-Adeno-1 plasmid and pLT-AAVhelp plasmid using the calcium phosphate method. Transfected cells were harvested after 56 h and repeatedly frozen and thawn. Benzonase was added to a final concentration of 250 U/ml and incubated for 30 min at 37 °C. After centrifugation at 8000 rpm for 10 min at 4 °C the supernatant was transferred to a new tube and deoxycholic acid (Sigma) was added to a final concentration of 0.5 % and incubated at 37 °C for 30 min. Viruses were then affinity purified and concentrated using heparin columns as reported elsewhere (Auricchio A, Hildinger M, O'Connor E, Gao GP, Wilson JM (2001) Isolation of highly infectious and pure adeno-associated virus type 2 vectors with a single-step gravity-flow column. Human Gene Therapy 12:71-76). Finally viral titers were estimated by means of AAV-HT1080 cells (Stratagene).

### Optic nerve crush, lens injury and administration of AAV-2

For all experiments adult female Sprague-Dawley rats, 220-250 g, were anesthetized by intraperitoneal injection of ketamine (60-80 mg/kg) and xylazine (10-15 mg/kg), and a 1-1.5 cm incision was made in the skin above the right orbit. The optic nerve was surgically exposed under an operating microscope. The epineurium was opened longitudinally, and the nerve was either cut or crushed 0.5 mm behind the eye for 10 seconds using a jeweler's forceps, avoiding injury to the ophthalmic artery. Optic nerve crush was verified by the appearance of a clearing at the lesion site. The vascular integrity of the retina was verified by fundoscopic examination. Sham surgery was performed accordingly without injuring the optic nerve. Lens injury was induced through a retrolenticular approach, puncturing the lens capsule with the narrow tip of a microcapillary tube; inflammation was enhanced by injecting 10 µl of phosphate buffered saline (PBS) intravitreally after retrieving the same volume from the anterior chamber of the eye.

For viral transfection of RGCs 15 µl of an AAV solution (∼10¹¹ particles) were intravitreally injected as described before. All surgical procedures were approved by the local authorities (Regierungspräsidium Tübingen).

### Retinal explant cultures

Five days after optical nerve crush (ONC) and lens injury (LI) or sham intraocular surgery, rats were killed and their retinas dissected (n = 4 animals per group). Isolated retinas were cut into eight radial pieces and cultured in DMEM containing B27-supplement (Gibco) (1:50) and 1:50 penicillin/streptomycin (Biochrom) in laminin-poly-D-lysine coated petriperm dishes (Greiner bio-one) (Bähr M, Vanselow J, Thanos S. In vitro regeneration of adult rat ganglion cell axons from retinal explants. Exp Brain Res. 1988; 73(2):393-401). After 2 days, explants were fixed with a paraformaldehyde solution (4 %) and methanol. Axons were then stained with a monoclonal antibody against βIII-tubulin (TUJ-1) (Babco, Richmond, CA; 1:2000). The number of axons extending ≥ 50 µm from each explant was counted using a fluorescent microscope (x 200, Axiovert, Carl Zeiss, Germany) and a calibrated ocular micrometer. The results from individual explants were averaged within each experimental group and differences between the groups were evaluated using the Student's t-test.

### Generation of the anti-LINA antibody

The anti-LINA antibody was generated in rabbits against the peptide NKPIAGPTETSQS at *Invitrogen.* The antibody was purified by affinity chromatography against the peptide according to standard protocols.

### Quantitation of axon regeneration in the optic nerve

Regeneration was quantified as described (Leon S, Yin Y, Nguyen J, Irwin N, Benowitz LI (2000) Lens injury stimulates axon regeneration in the mature rat optic nerve. J Neurosci 20:4615-4626; Fischer D, He Z, Benowitz LI (2004a) Counteracting the Nogo receptor enhances optic nerve regeneration if retinal ganglion cells are in an active growth state. J Neurosci 24:1646-1651). In brief, under 400 X magnification, we counted the number of GAP43-positive axons extending > 250 µm, > 500 µm and > 1 mm from the injury site in 8 sections per case, normalized these numbers to the cross-sectional width of the optic nerve, and used these data to calculate the total numbers of regenerating axons in each animal as described (Leon S, Yin Y, Nguyen J, Irwin N, Benowitz LI (2000) Lens injury stimulates axon regeneration in the mature rat optic nerve. J Neurosci 20:4615-4626; Fischer D, He Z, Benowitz LI (2004a) Counteracting the Nogo receptor enhances optic nerve regeneration if retinal ganglion cells are in an active growth state. J Neurosci 24:1646-1651). Each experimental group comprised at least 5 animals. The significances of intergroup differences were evaluated by Student's t-tests.

Quantitation of RGCs in cross-sections: Cross-sections through the center of the retina were stained with antibodies βIII-tubulin as described above. The numbers of βIII-tubulin-positive cells per section were counted in 4-6 sections per case, averaged for each case, and then averaged across all similarly treated animals to obtain group means and standard errors.

### Results

### Cloning of LINA

It was recently shown that the regenerative switch of RGCs to a regenerative state is associated with a change in gene expression. Among those were several expressed sequence tags (ESTs) representing uncharacterized or unknown proteins. One EST, which revealed a similarity to a so far uncharacterized mouse gene (RNF 122) (probe set ID: 1373485_at, Chip RAE230A) showed a 1.9 fold increase after ONC and a 3.6 fold increase after additional LI compared with untreated controls (Lee J, Hauk TG, Nonnenmacher L, Böckers TM, Benowitz LI and Fischer D. LINA: a new ring finger protein promoting axon regeneration, Abstract for annual Meeting of the Society of Neuroscience, 2006 in Atlanta). Using RT-PCR, the cDNA including the putative coding sequence of mouse, rat and human RNF 122. Rat RNF 122 that was named LINA consists of 155 amino acids was generated and sequenced. Amino acid sequences of rat and mouse RNF 122 were identical and human RNF 122 differed just in five amino acids at the c-terminus of the protein (Figure 1 A). Bioinformatic sequence analysis predicted a ring finger domain (C₃H₂C₃ type) between amino acids 93 and 133, a putative transmembrane domain between amino acids 37 and 59 and a glycosylation site at N20 (Figure 1A, B) .

Exogenously expressed HA-tagged-LINA ran in gels under denaturating conditions in two bands at 19 and 23 kDa (Figure 1 C), pointing to a posttranslational glycosylation of the protein. Incubation of LINA with N-glycosidase or a substitution of amino acid asparagine (N20) with glutamine (Q) eliminated the upper band, revealing that LINA is glycosylated at N20 (Fig. 1 C) and that the two bands represent the glycosylated and unglycosylated form of the protein.

### Subcellular localization of LINA in secretory vesicles

Both HA-tagged and GFP-tagged LINA-fusionproteins showed a granular staining pattern when expressed in HEK293 or PC12 cells with a partial colocalization with the Trans-Golgi-network marker TGN38 (Figure 2-I, C-F). In PC12 cells LINA was detected in the cell body and neurites. Time labs analysis revealed a localization of LINA in rapidly moving vesicles (supplementary data).

To detect endogenously expressed LINA we used an affinity purified peptide antibody against the C-terminus of the protein. The specificity of this affinity purified antibody was verified by Western-blot and immunocytochemical analysis and detected exogenous expressed LINA by both methods. Consistently with LINA-fusionproteins immunocytochemical staining of PC12 cells, cultured RGCs and embryonic hippocampal neurons showed a granular staining pattern with a pronounced accumulation in the TGN (Figure 2-I, A, B, C). In hippocampal neurons LINA was mainly detected in the cell bodies but also in dendrites and axons (Figure 2-I, A). Cultured RGCs showed little LINA in regenerating axons and cell bodies. Endogenous or exogenously expressed LINA was never seen in the plasma membrane or nucleus of cells (Figure 2-I, B, C) .

To further investigate the localization of LINA in the cell LINA-fusionproteins have been overexpressed in PC12 cells and subcellular markers have been used. LINA did not co-lolocalize with lysosomes, detected by lysotracker (Invitrogen) (Figure 2-II, A, B, C) or peroxisomes, which became detectable after overexpression of SKL-GFP fusionprotein (Figure 2-II, D, E, F). LINA partially colocalized with BACE1-GFP-fusionproteins, indicating that LINA is expressed in vesicles of the secretory pathway (Figure 2-II, G, H, I) .

### LINA is widely expressed in the nervous system

Various tissues for LINA expression in the adult rat were screened, using RT-PCR and Real-time PCR and in-situ-hybridization for embryonic tissue. In adult rats LINA-mRNA was detected in retina, cerebellum, spinal cord, brain cortex, dorsal root ganglia and spleen. Low expression levels of LINA were detected in skeletal muscle, heart, kidney and liver (Figure 3, B, C). *In situ*-hybridisation of embryonic rats (E16) showed LINA expression in the subventricular zone of the brain, cerebellum, retina and lung. LINA-probes did not significantly detect other embryonic organs (Figure 3, A).

### LINA expression is upregulated in regenerating RGCs

Retinal LINA expression was assessed by RT-PCR, *in-situ*-hybridization, Western-blot and immunohistochemistry:

RT-PCR detected LINA in naive retinas. Levels of the ring finger protein were slightly increased after ONC and elevated further after additional LI treatment confirming the expression results of our microarray analysis (Fischer D, Petkova V, Thanos S, Benowitz LI (2004b) Switching mature retinal ganglion cells to a robust growth state in vivo: gene expression and synergy with RhoA inactivation. J Neurosci 24:8726-8740) (Figure 4, L). LI without ONC increased LINA expression, too. *In situ*-hybridization using two distinct DNA-probes detected LINA in retinas that had been prior exposed to LI, whereas signals in untreated retinas were low to absent. Signals from radioactive probes accumulated in the RGC layer as determined by dipped sections (Figure 4, A, B, C).

For LINA detection on the protein level was used the peptide antibody against the c-terminus, which detected LINA as a single band of 21 kD in naive retinas at the same level as glycosylated exogenous expressed rat LINA in HEK293 cells in Western-blots (Figure 4, M). Expression levels were not significantly altered five days after ONC, but showed an increase after LI treatment either with or without ONC. Consistently immunohistochemical staining showed a weak staining of naive RGCs. Staining intensity slightly increased in a small percentage of RGCs five days after ONC. In these cases most LINA was located in the dendrites of RGCs. LI alone or combined with ONC strongly upregulated LINA expression in RGCs (Figure 4, D-K). RGCs showed a vesicular staining pattern with a pronounced staining of the cell body and dendrites. Axons in the fiber layer were only weakly stained with the antibody.

### LINA expression is essential for neurite outgrowth in PC12 cells and overexpression promotes outgrowth

In order to assess a potential a role of LINA in neurite outgrowth we initially used PC12 cells (pheochromocytoma cells). Untreated PC12 cells express LINA as determined by RT-PCR, immunocytochemistry and Western-blot analysis (Figure 5, A-D). PC12 cells differentiate and extended neurites in response to the nerve growth factor (NGF) that was added to the medium (50 ng/ml). Expression levels of LINA increased in differentiating PC12 cells after exposure to NGF about five-fold after 24 h as measured by quantitative RT-PCR. The increase of LINA expression in PC12 cells was verified on the protein level by immunocytochemistry and Western-blotting using an affinity purified peptide-antibody against LINA (Figure 5, A-E). Next to the cell body of PC12 cells LINA was also detected in the neurites and growth cones.

Next it was tested whether LINA expression affects neurite outgrowth of PC12 cells. Overexpression itself did not initiate neurite outgrowth and cells showed a round shape. However, when exposed to NGF LINA overexpression increased neurite outgrowth about 2.5 fold compared to controls transfected with an empty control plasmid. The neurite outgrowth promoting effect was dependent on an intact ring-finger domain. (Figure 5, F-I). This data suggest that endogenous LINA and its upregulation are essential for NGF induced neurite outgrowth of PC12 cells.

### LINA overexpressing promotes axon regeneration of mature RGCs

To investigate the role of LINA in primary RGCs in vivo and *in vitro* we generated Adeno Associated Viruses (AAV) for LINA^{WT} overexpression and a control virus carrying the empty plasmid. Because RGCs somata and axons are superficial in the retina intravitreal injections of Adeno Associated Viruses transfect ∼75% of RGCs but very few other cell types (DiPolo R, Beauge L (1998) Differential up-regulation of Na+-Ca2+ exchange by phosphoarginine and ATP in dialysed squid axons. J Physiol 507 (Pt 3):737-747; Fischer D, He Z, Benowitz LI (2004a) Counteracting the Nogo receptor enhances optic nerve regeneration if retinal ganglion cells are in an active growth state. J Neurosci 24:1646-1651; Fischer D, Petkova V, Thanos S, Benowitz LI (2004b) Switching mature retinal ganglion cells to a robust growth state in vivo: gene expression and synergy with RhoA inactivation. J Neurosci 24:8726-8740; Zhou Y, Pernet V, Hauswirth WW, Di Polo A (2005) Activation of the extracellular signal-regulated kinase 1/2 pathway by AAV gene transfer protects retinal ganglion cells in glaucoma. Mol Ther 12:402-412; Leaver SG, Cui Q, Plant GW, Arulpragasam A, Hisheh S, Verhaagen J, Harvey AR (2006) AAV-mediated expression of CNTF promotes long-term survival and regeneration of adult rat retinal ganglion cells. Gene Ther 13:1328-1341).

The high efficiency and specificity of transfection was verified by double labeling studies showing the GFP reporter was expressed in the same cells that expressed the RGCs-specific tubulin isoform βIII tubulin three weeks after virus injection. The expression of the transgenes in RGCs was further verified with antibodies detecting the HA-tag of LINA proteins (Figure 6, A-I).

To elucidate whether LINA overexpression would affect axon regeneration we crushed optic nerves three weeks after intravitreal virus application and explanted retinas on poly-L-lysin/laminin coated dishes five days later. The number and length of axons extending from explants were quantified after being two days in culture. Overexpression of LINA^{WT} in RGCs increased the number of outgrowing axons 2.5 fold compared to controls transfected with a virus carrying the empty plasmid. The percentage of long axons extending 0.5 mm was three fold higher in LINA overexpressing cases compared to controls, suggesting that LINA overexpression facilitated axon regeneration (Figure 6, J, K, L, M).

### LINA overexpressing enhances RGC survival and promotes axon regeneration of into the injured optic nerve

To investigate whether LINA overexpression would also affect axon regeneration *in vivo* the optic nerves have been crushed three weeks after viral transduction of RGCs. Regenerating axons were identified by an antibody against GAP43 two weeks after surgery (Figure 7, A-D). The number of axons regenerating ≥ 0.5 mm and ≥ 1 mm were quantified as described before (Fischer D, He Z, Benowitz LI (2004a) Counteracting the Nogo receptor enhances optic nerve regeneration if retinal ganglion cells are in an active growth state. J Neurosci 24:1646-1651; Fischer D, Petkova V, Thanos S, Benowitz LI (2004b) Switching mature retinal ganglion cells to a robust growth state in vivo: gene expression and synergy with RhoA inactivation. J Neurosci 24:8726-8740).

At first, the influence of LINA^{WT} overexpression in the absence of LI was investigated. Consistently with earlier results controls transduced with the control virus showed only very few axons regenerating ≥ 0.5 mm and no axons extending ≥ lmm beyond the lesion site. In comparison to this control group LINA^{WT} overexpressing caused approximately four times more axons regenerating beyond ≥ 0.5 mm (p < 0.001) and in average 40 axons per optic nerve extending ≥ 1 mm. Adjacent retinal sections of the same eyes were immunostained with an antibody against βIII-tubulin and the number of surviving RGCs per section determined for each group as described before (Fischer D, He Z, Benowitz LI (2004a) Counteracting the Nogo receptor enhances optic nerve regeneration if retinal ganglion cells are in an active growth state. J Neurosci 24:1646-1651; Fischer D, Petkova V, Thanos S, Benowitz LI (2004b) Switching mature retinal ganglion cells to a robust growth state in vivo: gene expression and synergy with RhoA inactivation. J Neurosci 24:8726-8740). LINA^{WT} overexpression increased also the survival of RGCs by 46 % compared to controls (Figure 7, F). However these neuroprotective effects were much lower than observed after LI.

Since LINA^{WT} overexpression enhanced axon regeneration much stronger than RGC survival it was tested next whether LINA^{WT} could increase axon regeneration further when combined with the strong neuroprotective effects of LI. Two weeks after nerve crush and LI, animals expressing LINA^{WT} extended 2.1 times more axons > 1 mm beyond the injury site than LI treated controls expressing GFP alone. The survival of RGCs in LINA^{WT} overexpressing retinas exposed to LI was about 23 % increased compared to controls expressing just GFP and treated with LI only (Figure 7, C-G).

## Claims

1. Use of a polypeptide comprising at least the ring finger domain of RING finger protein 122, of a recombinant vector comprising DNA or RNA coding at least for the ring finger domain of RNF 122 and/or of a recombinant organism comprising DNA or RNA coding at least for the ring finger domain of RNF 122 in medicine.

2. Use of a polypeptide comprising at least the ring finger domain of RING finger protein 122, of a recombinant vector comprising DNA or RNA coding at least for the ring finger domain of RNF 122 and/or of a recombinant organism comprising DNA or RNA coding at least for the ring finger domain of RNF 122 according to claim 1 as a medicinal drug.

3. Use of a polypeptide comprising at least the ring finger domain of RING finger protein 122, of a recombinant vector comprising DNA or RNA coding at least for the ring finger domain of RNF 122 and/or of a recombinant organism comprising DNA or RNA coding at least for the ring finger domain of RNF 122 for manufacturing of a medicinal drug for treatment of at least one of the diseases of the group comprising diseases of the nervous system, in particular of the central nervous system; ocular diseases, in particular glaucoma, intraocular inflammation, optic nerve injuries, in particular crushed optic nerves; neuronal diseases, in particular injured axons; traumatic injuries of the nervous system; stroke; diseases of spinal cord and diseases after an apoplexy.

4. Use of a polypeptide comprising at least the ring finger domain of RING finger protein 122, of a recombinant vector comprising DNA or RNA coding at least for the ring finger domain of RNF 122 and/or of a recombinant organism comprising DNA or RNA coding at least for the ring finger domain of RNF 122 for manufacturing of a medicinal drug for treatment of ocular diseases by intravitreal injection of the medicinal drug.

5. Use of a recombinant vector comprising DNA or RNA coding at least for the ring finger domain of RNF 122 and/or of a recombinant organism comprising DNA or RNA coding at least for the ring finger domain of RNF 122 according to one of the previous claims, **characterized in that** the recombinant vector and/or the recombinant organism comprises DNA or RNA of mouse origin or DNA or RNA of rat origin or DNA or RNA of human origin.

6. Use of a recombinant vector comprising DNA or RNA coding at least for the ring finger domain of RNF 122 and/or of a recombinant organism comprising DNA or RNA coding at least for the ring finger domain of RNF 122 according to one of claims 1 to 5, **characterized in that** the recombinant vector and/or the recombinant organism comprises at least the following RNA nucleotide sequence coding for at least the ring finger domain of RNF 122:

acacgucaaacagaccuucugaag
uuccccuuccuacuugauccacacgaaggc
acggucguacggaaaguggcguucacagac
cacuucaccgaucuucacgcaacgcagacg
ggguacacg.

7. Use of a recombinant vector comprising DNA or RNA coding at least for the ring finger domain of RNF 122 and/or of a recombinant organism comprising DNA or RNA coding at least for the ring finger domain of RNF 122 according to claim 6, **characterized in that** the recombinant vector and/or the recombinant organism comprises at least the following RNA nucleotide sequence:

uacgugguaaggucaccacauugcccaca
aagacaccggaccccgaccacucaugguug
uucaggacgaguuacgugguuagucaaag
guccuggaaggggaguuguagauguaccag
uagaagccgugaccguagaaacagaaauac
gagucagaguagaagacgacgaugaaguag
ucguuugaggccuugguccgugucucgcuc
gcuaugccgauguuccuccaccacgaauuu
ccacuacgauucuucaaugucgagauaccc
gucugg acacgucaaacagaccuucugaag
uuccccuuccuacuugauccacacgaaggc
acggucguacggaaaguggcguucacagac
cacuucaccgaucuucacgcaacgcagacg
ggguacacg uuguucggguaacgaccgggg
ugccucuggagcgucucguaacccuaggac
gaccuacuuaaccacauu.

8. Use of a recombinant vector comprising DNA or RNA coding at least for the ring finger domain of RNF 122 and/or of a recombinant organism comprising DNA or RNA coding at least for the ring finger domain of RNF 122 according to one of claims 1 to 5, **characterized in that** the recombinant vector and/or the recombinant organism comprises at least the following DNA nucleotide sequence:

tgtgcagtttgtctggaagacttc
aaggggaaggatgaactaggtgtgcttccg
tgccagcatgcctttcaccgcaagtgtctg
gtgaagtggctagaagtgcgttgcgtctgc
cccatgtgc.

9. Use of a recombinant vector comprising DNA or RNA coding at least for the ring finger domain of RNF 122 and/or of a recombinant organism comprising DNA or RNA coding at least for the ring finger domain of RNF 122 according to claim 8, **characterized in that** the recombinant vector and/or the recombinant organism comprises at least the following DNA nucleotide sequence:

atgcacccattccagtggtgtaacggtgt
ttctgtggcctggggctggtgagtaccaac
aagtcctgctcaatgccaccaatcagtttc
caggaccttcccctcaacatctacatggtc
atcttcggcactggcatctttgtctttatg
ctcagtctcatcttctgctgctacttcatc
agcaaactccggaaccaggcacagagcgag
cgatacggctacaaggaggtggtgcttaaa
ggtgatgctaagaagttacagctctatggg
cagacc tgtgcagtttgtctggaagacttc
aaggggaaggatgaactaggtgtgcttccg
tgccagcatgcctttcaccgcaagtgtctg
gtgaagtggctagaagtgcgttgcgtctgc
cccatgtgc aacaagcccattgctggcccc
acggagacctcgcagagcattggatcctg

ctggatgaattggtgtaa.

10. Use of a polypeptide according to one of claims 1 to 4, **characterized in that** the polypeptide comprises at least or consists of the following amino acid sequence: CAVCLEDF KGKDELGVLP CQHAFHRKCL VKWLEVRCVR PMC.

11. Use of a polypeptide according to claim 10, **characterized in that** the polypeptide comprises or consists of the following amino acid sequence: 
MHPFQWCNGC FCGLGLVSTN KSCSMPPISF QDLPLNIYMV
IFGTGIFVFM LSLIFCCYFI SKLRNQAQSE RYGYKEVVLK
GDAKKLQLYG QTCAVCLEDF KGKDELGVLP CQHAFHRKCL
VKWLEVRCVR PMCNKPIA*P *E**Q*IGIL LDELV,
 wherein * indicates a not specified amino acids.

12. Use of a polypeptide according to the previous claim, **characterized in that** the amino acid sequence comprises as amino acid 139 G or *S and*/*or* as amino acid 141 T or S *and*/*or* as amino acid 143 T or A *and*/*or* as amino acid 144 S or T *and*/*or* as amino acid 146 S or N.

13. Use of a polypeptide comprising at least the ring finger domain of RING finger protein 122, of a recombinant vector comprising DNA or RNA coding at least for the ring finger domain of RNF 122 and/or of a recombinant organism comprising DNA or RNA coding at least for the ring finger domain of RNF 122 according to one of claims 1 to 12, **characterized in that** the polypeptide, the recombinant vector and/or the recombinant organism is used in combination with a nerve growth factor (NGF) or ciliary neurotrophic factor (CNTF) and/or axon growth promoting factors.

14. RNA with the following nucleotide sequence:

uacgugguaaggucaccacauugcccaca
aagacaccggaccccgaccacucaugguug
uucaggacgaguuacgguguuagucaaag
guccuggaaggggaguuguagauguaccag
uagaagccgugaccguagaaacagaaauac
gagucagaguagaagacgacgaugaaguag
ucguuugaggccuugguccgugucucgcuc
gcuaugccgauguuccuccaccacgaauuu
ccacuacgauucuucaaugucgagauaccc
gucugg acacgucaaacagaccuucugaag
uuccccuuccuacuugauccacacgaaggc
acggucguacggaaaguggcguucacagac
cacuucaccgaucuucacgcaacgcagacg
ggguacacg uuguucggguaacgaccgggg
ugccucuggagcgucucguaacccuaggac
gaccuacuuaaccacauu.

15. DNA with the following nucleotide sequence:

atgcacccattccagtggtgtaacgggtgt
ttctgtggcctggggctggtgagtaccaac
aagtcctgctcaatgccaccaatcagtttc
caggaccttcccctcaacatctacatggtc
atcttcggcactggcatctttgtctttatg
ctcagtctcatcttctgctgctacttcatc
agcaaactccggaaccaggcacagagcgag
cgatacggctacaaggaggtggtgcttaaa
ggtgatgctaagaagttacagctctatggg
cagacc tgtgcagtttgtctggaagacttc

aaggggaaggatgaactaggtgtgcttccg
tgccagcatgcctttcaccgcaagtgtctg
gtgaagtggctagaagtgcgttgcgtctgc
cccatgtgc aacaagcccattgctggcccc
acggagacctcgcagagcattgggatcctg
ctggatgaattggtgtaa.

16. Polypeptide, **characterized in that** it is produced using the RNA of claim 14 or the DNA of claim 15.

17. Rat polypeptide comprising or consisting of the following amino acid sequence:

MHPFQWCNGC FCGLGLVSTN KSCSMPPISF QDLPLNIYMV
IFGTGIFVFM LSLIFCCYFI SKLRNQAQSE RYGYKEVVLK
GDAKKLQLYG QTCAVCLEDF KGKDELGVLP CQHAFHRKCL
VKWLEVRCVR PMCNKPIAGP TETSQSIGIL LDELV.

18. Recombinant vector, **characterized in that** it contains RNA according to claim 14, DNA according to claim 15 or DNA or RNA which codes for a polypeptide according to claim 17.

19. Recombinant vector according to the previous claim, **characterized in that** it is a plasmid, preferably an AAV-MCS2-IGFP plasmid or another AAV expression vector.

20. Recombinant organism, **characterized in that** it has at least one of the following characteristics a) to d)
a) it has been transformed with a vector according to claim 18 or 19;
b) it comprises at least a sequence of DNA or RNA which codes for a polypeptide according to claim 17;
c) it is a virus, preferably an adeno associated virus (AAV); and
d) it consists of cells, preferably of human embryonic cells (HEC), pheochromocytoma cells (PC12) or retinal ganglion cells (RGC).

21. Method of a medical treatment, **characterized in that** a polypeptide comprising at least the ring finger domain of RING finger protein 122, a recombinant vector comprising DNA or RNA coding at least for the ring finger domain of RNF 122 and/or a recombinant organism comprising DNA or RNA coding at least for the ring finger domain of RNF 122 is applied to an animal, in particular a mammal, in particular a human being.

22. Method according to claim 21 for the treatment of at least one of the diseases of the group comprising diseases of the nervous system, in particular of the central nervous system; ocular diseases, in particular retinal diseases, intraocular inflammation, optic nerve injuries, in particular crushed optic nerves; glaucoma; neuronal diseases, in particular injured axons; traumatic injuries of the nervous system; stroke; diseases of spinal cord and diseases after an apoplexy.


SEQUENCE LISTING

<110>  Fischer, Dietmar

<120>  Protein with promoting effects for axonal growth of neurons of
       central nervous system

<130>  089EP 1544

<160>  21

<170>  PatentIn version 3.3

<210>  1
<211>  28
<212>  DNA
<213>  artificial

<220>
<223>  Primer for RT-PCR forward

<400>  1
cattgatgca cccattccag tggtgtaa                                          28


<210>  2
<211>  25
<212>  DNA
<213>  artificial

<220>
<223>  Primer for RT-PCR reverse

<400>  2
gcaaggagcg cttacaccaa ttcat                                             25


<210>  3
<211>  32
<212>  DNA
<213>  artificial

<220>
<223>  PCR Primer for cloning cDNA forward

<400>  3
gtcaaattga attcccttga ctccactcct cc                                     32


<210>  4
<211>  32
<212>  DNA
<213>  artificial

<220>
<223>  PCR Primer for cloning cDNA reverse

<400>  4
gttatattct cgaggcaagg agcgcttaca cc                                     32


<210>  5
<211>  65
<212>  DNA
<213>  artificial

<220>
<223>  Reverse Primer for generating C-terminal HA-tagged LINA

<400>  5
atattctcga gttaagcata atctggaaca tcatatggat acatcaccaa ttcatccagc      60

aggat                                                                  65


<210>  6
<211>  39
<212>  DNA
<213>  artificial

<220>
<223>  Antisense Oligonucleotide

<400>  6
gccacttcac cagacacttg cggtgaaagg catgctggc                             39


<210>  7
<211>  38
<212>  DNA
<213>  artificial

<220>
<223>  Antisense Oligonucleotide 2

<400>  7
gcaatgggct tgttgcacat ggggcagacg caacgcac                              38


<210>  8
<211>  19
<212>  DNA
<213>  artificial

<220>
<223>  Forward Primer for Detection of LINA

<400>  8
ccattccagt ggtgtaacg                                                   19


<210>  9
<211>  18
<212>  DNA
<213>  artificial

<220>
<223>  Reverse Primer for Detection of LINA

<400>  9
agatgccagt gccgaaga                                                    18


<210>  10
<211>  28
<212>  DNA
<213>  artificial

<220>
<223>  Primer Semi-quantitative RT-PCR LINA

<400>  10
cattgatgca cccattccag tggtgtaa                                         28


<210>  11

<211>  25
<212>  DNA
<213>  artificial

<220>
<223>  Reverse Primer Semi-quantitaive RT-PCR LINA

<400>  11
gcaaggagcg cttacaccaa ttcat                                          25


<210>  12
<211>  24
<212>  DNA
<213>  artificial

<220>
<223>  Forward Primer GAPDH

<400>  12
ggccaaggtc atccatgaca actt                                           24


<210>  13
<211>  24
<212>  DNA
<213>  artificial

<220>
<223>  Reverse Primer GAPDH

<400>  13
cctgcttcac caccttcttg atgt                                           24


<210>  14
<211>  13
<212>  PRT
<213>  artificial

<220>
<223>  Peptide for generating an anti-LINA antibody

<400>  14

Asn Lys Pro Ile Ala Gly Pro Thr Glu Thr Ser Gln Ser
1               5                   10


<210>  15
<211>  123
<212>  RNA
<213>  artificial

<220>
<223>  RNA nucleotide sequence

<400>  15
acacgucaaa cagaccuucu gaaguucccc uuccuacuug auccacacga aggcacgguc    60

guacggaaag uggcguucac agaccacuuc accgaucuuc acgcaacgca gacgggguac    120

acg                                                                 123


<210>  16
<211>  468
<212>  RNA

<213>  artificial

<220>
<223>  Nucleotide sequence

<400>  16
uacgugggua aggucaccac auugcccaca aagacaccgg accccgacca cucaugguug      60

uucaggacga guuacggugg uuagucaaag guccuggaag gggaguugua gauguaccag     120

uagaagccgu gaccguagaa acagaaauac gagucagagu agaagacgac gaugaaguag     180

ucguuugagg ccuugguccg ugucucgcuc gcuaugccga uguuccucca ccacgaauuu     240

ccacuacgau ucuucaaugu cgagauaccc gucuggacac gucaaacaga ccuucugaag     300

uuccccuucc uacuugaucc acacgaaggc acggucguac ggaaaguggc guucacagac     360

cacuucaccg aucuucacgc aacgcagacg ggguacacgu uguucgggua acgaccgggg     420

ugccucugga gcgucucgua acccuaggac gaccuacuua accacauu                  468

<210>  17
<211>  123
<212>  DNA
<213>  artificial

<220>
<223>  DNA nucleotide sequence

<400>  17
tgtgcagttt gtctggaaga cttcaagggg aaggatgaac taggtgtgct tccgtgccag      60

catgcctttc accgcaagtg tctggtgaag tggctagaag tgcgttgcgt ctgccccatg     120

tgc                                                                   123

<210>  18
<211>  468
<212>  DNA
<213>  artificial

<220>
<223>  DNA nucleotide sequence

<400>  18
atgcacccat tccagtggtg taacgggtgt ttctgtggcc tggggctggt gagtaccaac      60

aagtcctgct caatgccacc aatcagtttc caggaccttc ccctcaacat ctacatggtc     120

atcttcggca ctggcatctt tgtctttatg ctcagtctca tcttctgctg ctacttcatc     180

agcaaactcc ggaaccaggc acagagcgag cgatacggct acaaggaggt ggtgcttaaa     240

ggtgatgcta agaagttaca gctctatggg cagacctgtg cagtttgtct ggaagacttc     300

aaggggaagg atgaactagg tgtgcttccg tgccagcatg cctttcaccg caagtgtctg     360

gtgaagtggc tagaagtgcg ttgcgtctgc cccatgtgca acaagcccat tgctggcccc     420

acggagacct cgcagagcat tgggatcctg ctggatgaat tggtgtaa                  468

<210>  19
<211>  41
<212>  PRT

<213>  artificial

<220>
<223>  amino acid sequence of a polypeptide

<400>  19

Cys Ala Val Cys Leu Glu Asp Phe Lys Gly Lys Asp Glu Leu Gly Val
1               5                   10                  15

Leu Pro Cys Gln His Ala Phe His Arg Lys Cys Leu Val Lys Trp Leu
            20                  25                  30

Glu Val Arg Cys Val Arg Pro Met Cys
        35                  40

<210>  20
<211>  155
<212>  PRT
<213>  artificial

<220>
<223>  Amino acid sequence

<220>
<221>  special feature
<222>  (139)..(139)
<223>  Xaa is Gly or Ser

<220>
<221>  special feature
<222>  (141)..(141)
<223>  Xaa is Thr or Ser

<220>
<221>  special feature
<222>  (143)..(143)
<223>  Xaa is Thr or Ala

<220>
<221>  special feature
<222>  (144)..(144)
<223>  Xaa is Ser or Thr

<220>
<221>  special feature
<222>  (146)..(146)
<223>  Xaa is Ser or Asn

<400>  20

Met His Pro Phe Gln Trp Cys Asn Gly Cys Phe Cys Gly Leu Gly Leu
1               5                   10                  15

Val Ser Thr Asn Lys Ser Cys Ser Met Pro Pro Ile Ser Phe Gln Asp
            20                  25                  30

Leu Pro Leu Asn Ile Tyr Met Val Ile Phe Gly Thr Gly Ile Phe Val
        35                  40                  45

Phe Met Leu Ser Leu Ile Phe Cys Cys Tyr Phe Ile Ser Lys Leu Arg

50                  55                  60

Asn Gln Ala Gln Ser Glu Arg Tyr Gly Tyr Lys Glu Val Val Leu Lys
65                  70                  75                  80

Gly Asp Ala Lys Lys Leu Gln Leu Tyr Gly Gln Thr Cys Ala Val Cys
                85                  90                  95

Leu Glu Asp Phe Lys Gly Lys Asp Glu Leu Gly Val Leu Pro Cys Gln
            100                 105                 110

His Ala Phe His Arg Lys Cys Leu Val Lys Trp Leu Glu Val Arg Cys
        115                 120                 125

Val Arg Pro Met Cys Asn Lys Pro Ile Ala Xaa Pro Xaa Glu Xaa Xaa
    130                 135                 140

Gln Xaa Ile Gly Ile Leu Leu Asp Glu Leu Val
145                 150                 155

<210>  21
<211>  155
<212>  PRT
<213>  artificial

<220>
<223>  Rat polypeptide

<400>  21

Met His Pro Phe Gln Trp Cys Asn Gly Cys Phe Cys Gly Leu Gly Leu
1               5                   10                  15

Val Ser Thr Asn Lys Ser Cys Ser Met Pro Pro Ile Ser Phe Gln Asp
            20                  25                  30

Leu Pro Leu Asn Ile Tyr Met Val Ile Phe Gly Thr Gly Ile Phe Val
        35                  40                  45

Phe Met Leu Ser Leu Ile Phe Cys Cys Tyr Phe Ile Ser Lys Leu Arg
    50                  55                  60

Asn Gln Ala Gln Ser Glu Arg Tyr Gly Tyr Lys Glu Val Val Leu Lys
65                  70                  75                  80

Gly Asp Ala Lys Lys Leu Gln Leu Tyr Gly Gln Thr Cys Ala Val Cys
                85                  90                  95

Leu Glu Asp Phe Lys Gly Lys Asp Glu Leu Gly Val Leu Pro Cys Gln
            100                 105                 110

His Ala Phe His Arg Lys Cys Leu Val Lys Trp Leu Glu Val Arg Cys
        115                 120                 125

Val Arg Pro Met Cys Asn Lys Pro Ile Ala Gly Pro Thr Glu Thr Ser
    130                 135                 140

Gln Ser Ile Gly Ile Leu Leu Asp Glu Leu Val
145                 150                 155